# EUROPÄISCHE PATENTSCHRIFT

(11) **EP 1 986 542 B1**
(45) Veröffentlichungstag und Bekanntmachung des Hinweises auf die Patenterteilung: **12.10.2011**
(21) Anmeldenummer: 07711600.2
(22) Anmeldetag: 21.02.2007
(51) Int. Cl.: A61B 1/24

(54) **DENTALES HANDSTÜCK**
DENTAL HANDPIECE
POIGNEE DENTAIRE

(30) Priorität: 24.02.2006 DE 102006009177
(43) Veröffentlichungstag der Anmeldung: 05.11.2008
(73) Patentinhaber: Dürr Dental AG, 74321 Bietigheim-Bissingen (DE)
(72) Erfinder: LAIS, Peter, 74391 Erligheim (DE); MAIER, Raimund, 71732 Tamm (DE)
(74) Vertreter: Ostertag, Reinhard
(86) Internationale Anmeldenummer: PCT/EP2007/001469
(87) Internationale Veröffentlichungsnummer: WO 2007/098874

(56) Entgegenhaltungen:
- FR-A1- 2 866 799
- JP-A- 2004 033 333
- US-A- 3 463 990
- US-A1- 2002 049 464

## Beschreibung

Die Erfindung betrifft ein dentales Handstück, insbesondere ein intraorales Kamerahandstück gemäß dem Oberbegriff des Anspruches 1.

Derartige Handstücke sind mit unterschiedlichen internen Arbeitskomponenten bekannt, z. B. Turbinen-Handstücke, Ultraschall-Handstücke, Spray-Handstücke und Kamera-Handstücke.

Derartige Handstücke beinhalten oft ein Betätigungselement, mit dem das Arbeiten des Handstückes gesteuert wird, z.B. einen Schalter zum Steuern der Sprühflüssigkeit bei einem Spray-Handstück.

Bei Betätigungselementen von Handstücken besteht immer die Gefahr, daß dort kontaminierte Flüssigkeit in das Innere des Handstückes eindringt. Dieses wird zwar nach jedem Gebrauch sterilisiert oder desinfiziert. Sterilisierung und Sterilisation können aber an solchen Stellen beeinträchtigt sein, die nur schlecht zugänglich sind, z.B. an Spalten. Auch ist für viele Arbeitskomponenten, insbesondere elektronische Komponenten, ein Zutritt von Flüssigkeiten, Feuchtigkeitsnebeln oder feuchter Luft unerwünscht.

Ein Handstück gemäβ dem Oberbegriff des Anspruchs 1 ist aus US-2002/049464 bekannt.

Durch die vorliegende Erfindung soll daher ein Handstück gemäß dem Oberbegriff des Anspruches 1 so weitergebildet werden, daß die Betätigungsmöglichkeit einer Arbeitskomponente gegeben ist, ohne daß ein Leckweg zwischen dem Gehäuseinneren und der Umgebung geschaffen wird, und zwar derart, dass eine besonders hohe Empfindlichkeit des Verformungssensors erhalten.

Diese Aufgabe ist erfindungsgemäß gelöst durch ein Handstück mit dem im Anspruch 1 angegebenen Merkmalen.

Bei dem erfindungsgemäßen Handstück ist das Gehäuse auch im Bereich der Betätigungseinrichtung absolut durchgehend. Es besteht somit dort keine Gefahr des Eindringens von Verunreinigungen.

Die Nähe der Betätigungseinrichtung zu der das Handstück führenden Hand erlaubt es, die zu steuernde Arbeitkomponente ergonomisch günstig und bequem ein- und auszuschalten bzw. zu triggern.

Bei einem erfindungsgemäßen Handstück kann die Betätigung an unterschiedlichen Stellen des ringförmigen Betätigungsabschnittes des Gehäuses erfolgen. Man kann also die Arbeitskomponente in gleicher Weise steuern, ganz gleich, in welcher Winkelorientierung das Handstück gerade gehalten wird.

Ein erfindungsgemäßes Handstück zeichnet sich auch durch besonders einfachen und robusten Aufbau aus. Falls gewünscht, kann man das analoge Ausgangssignal des Verformungssensors auch zu einer quantitativen Steuerung des Arbeitens einer Arbeitskomponente verwenden, z.B. zum Scharfstellen des Bildes einer Kamera.

Vorteilhafte Weiterbildungen der Erfindung sind in den Unteransprüchen angegeben.

Bei einem Handstück gemäß Anspruch 2 kann man das gesamte Gehäuse aus demselben Material herstellen. Die Verformbarkeit des der Betätigungseinrichtung zugeordneten Wandabschnittes des Gehäuses erhält man durch lokale Verkleinerung der Wanddicke.

Die Weiterbildung der Erfindung gemäß Anspruch 3 ist im Hinblick auf einfache und preisgünstige Herstellung von Vorteil. Kunststoff-Formteile zeichnen sich auch durch gute Oberflächenqualität und ansprechendes Äußeres aus

Bei einem Handstück gemäß Anspruch 4 kann man mehrere Arbeitskomponenten auf kleinem Raum bequem steuern. Die verschiedenen hinter dem verformbaren Wandabschnitt angeordneten Verformungssensoren können sich bezüglich ihrer Winkelstellung bezogen auf die Achse des Handstückes und/oder bezüglich ihrer axialen Stellung auf dem Handstück unterscheiden.

Mit der Weiterbildung der Erfindung gemäß Anspruch 6 erhält man ein Handstück, welches bei Betätigung eine Aktion auslösen kann, z.B. das Aufnehmen eines Bildes durch einen Bildwandler einer dentalen intraoralen Kamera.

Nachstehend wird die Erfindung anhand eines Ausführungsbeispieles unter Bezugnahme auf die Zeichnung näher erläutert. In dieser zeigen:
- Figur 1: eine seitliche Ansicht eines intraoralen dentalen Kamera-Handstückes;
- Figur 2: einen axialen Schnitt durch das Gehäuse des in Figur 1 gezeigten Handstückes;
- Figur 3: eine vergrößerte Ansicht der Anbringung eines Verformungssensorringes auf dem in Figur 2 gezeigten Gehäuse;
- Figur 4: eine abgewickelte Ansicht eines Piezofolien-Verformungssensors von einer Leiterseite her;
- Figur 5: eine Aufsicht auf den Piezofolien-Verformungssensors nach Figur 4 von der entgegengesetzten Seite her; und
- Figur 6: das Schaltbild eines Signalverarbeitungskreises, der in dem in Figur 1 gezeigten Handstück verwendet wird.

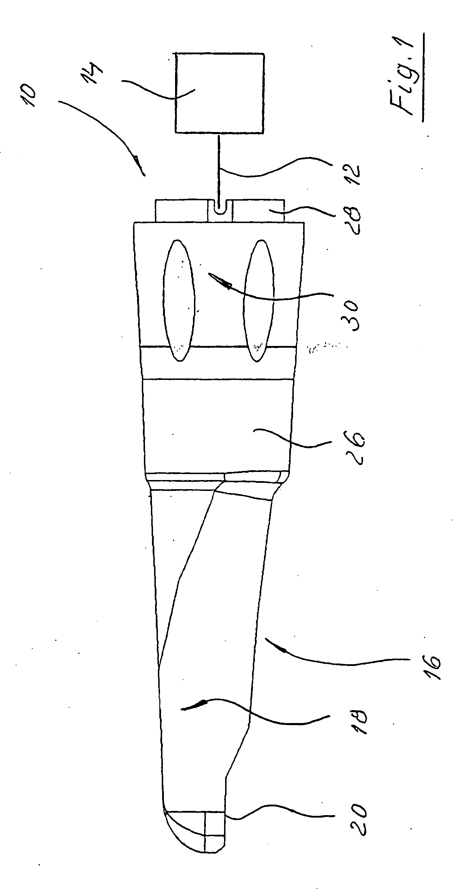

In Figur 1 ist mit 10 insgesamt ein dentales Kamera-Handstück bezeichnet. Dieses ist über ein nur schematisch angedeutetes Kabel 12 mit einer Betriebseinheit 14 versehen, welche Beobachtungslicht sowie Versorgungsspannungen für die im Kamerahandstück 10 enthaltenen elektronischen Komponenten (Bildwandler und Bildaufbereitung) bereitstellt und die vom Bildwandler der Kamera bereitgestellten Signale entgegennimmt und verarbeitet.

Das Kamerahandstück 10 hat ein insgesamt mit 16 bezeichnetes Gehäuse, welches ein Kunststoff-Spritzteil ist.

Das Gehäuse 16 umfasst ein vorderes langes Gehäuseteil 18, welches grob die Form eines schlanken Kegels hat und auf seiner Innenseite mehrfach abgestuft ist, um verschiedene optische Komponenten und den Bildwandler in vorgegebener Position aufnehmen zu können. Diese internen Arbeitskomponenten der Kamera sind der Übersichtlichkeit halber in der Zeichnung nicht wiedergegeben. Ein transparentes Fenster 20 des Gehäuseteiles 18 ermöglicht die Beobachtung im Mundraum.

Im Gehäuseteil 18 ist eine axiale Aufnahme 22 vorgesehen, in welcher ein Lichtleiter (nicht gezeigt) Aufnahme findet, der Licht durch das Fenster 20 an den zu beobachtenden Ort abgibt.

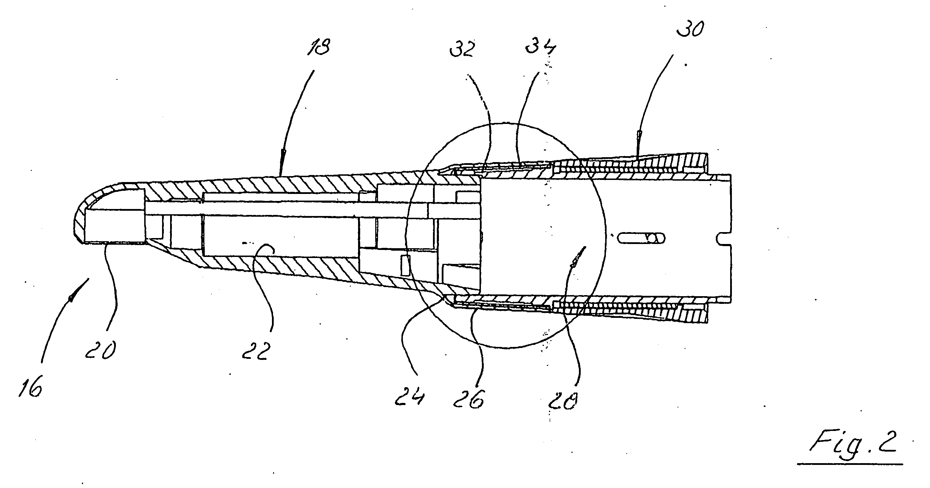

Im in Figur 2 links gelegenen Endabschnitt ist das Gehäuseteil 18 über einen konischen Wandabschnitt 24 mit einem hülsenförmigen Wandabschnitt 26 verbunden. Dieser hat verglichen mit der rechts von ihm gelegenen Wand des Gehäuseteiles 18 geringe Wandstärke und ist somit in radialer Einwärtsrichtung durch Fingerdruck verformbar.

Der hülsenförmige Wandabschnitt 26 überdeckt den in Figur 2 rechts gelegenen Endabschnitt einer Stützhülse 28, die zugleich dazu dient, eine Kupplungshülse 30 zu lagern, die zur lösbaren Verbindung und Verriegelung des Handstückes 10 mit dem entsprechend ausgebildeten Ende des Kabels 12 dient.

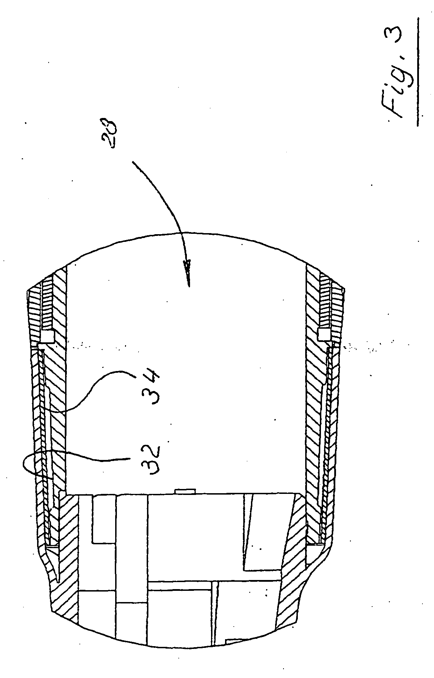

Wie insbesondere aus Figur 3 ersichtlich, hat die Stützhülse 28 in dem hinter dem hülsenförmigen Wandabschnitt 26 liegenden Teil eine flache Umfangsnut 32, die von einer Foliensensor 34 überdeckt wird.

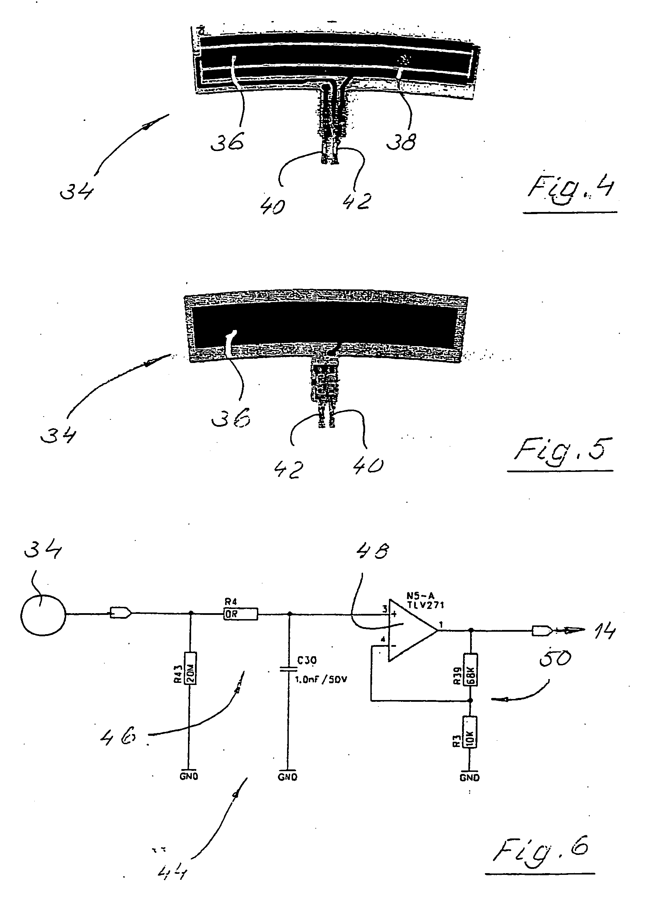

Der Foliensensor 34 ist beim betrachteten Ausführungsbeispiel eine Piezofolie, wie nachstehend unter Bezugnahme auf die Figuren 4 und 5 noch genauer beschrieben wird.

Die Geometrie des Foliensensors 34 ist so gewählt, daß er die Umfangsnut 32 und die dieser benachbarten Abschnitte der Außenfläche der Stützhülse 28 faltenfrei überdeckt. Beim hier betrachteten Ausführungsbeispiel weist der der Umfangsnut 32 benachbarte Bereich der Außenfläche der Kupplungshülse 30 eine schwache Konizität auf, so daß die Abwicklung des Foliensensors 34 der Abwicklung eines Kegelstumpfes entspricht, also durch zwei radial beabstandete Kreisbögen und zwei Abschnitte radialer Strahlen begrenzt ist.

Wie aus den Figuren 4 und 5 ersichtlich, besteht der Foliensensor 34 aus einer Sensorfolie 36 sowie auf diese aufgedruckten Leiterbahnen 38. Die Sensorfolie 36 ist aus piezoelektrischem Material hergestellt und die Leiterbahnen 38 bilden die Platten eines flächigen Kondensators. Wird die Sensorfolie 36 verformt, erhält man an Anschlußklemmen 40, 42 des Foliensensors 34 eine Spannung, die für die Verformung der Sensorfolie 36 charakteristisch ist.

Der Foliensensor 34 stellt einen mit piezoelektrischem Dielektrikum gefüllten Kondensator dar und erzeugt ein hochohmiges Ausgangssignal bereit. Um dieses in ein niederohmiges Signal umszusetzen, kann man den in Figur 6 gezeigten Signalverarbeitungskreis 44 (Impedanzwandler) einsetzen.

Die vom Foliensensor 34 abgegebene Spannung wird über ein hochohmiges RC-Filter 46 auf den einen positiven Eingang eines Operationsverstärkers 48 gegeben. Dessen Ausgangssignal wird über einen Spannungsteiler 50 auf den invertierenden Verstärkereingang zurückgeführt.

Das Ausgangssignal des Operationsverstärkers 48 ist eine Impedanz, deren Betrag der Verformung der Sensorfolie 36 entspricht und wird zur Weiterverarbeitung auf die Betriebseiheit 14 gegeben.

Das oben beschriebene Handstück wird wie folgt verwendet:
Durch Einschalten der Betriebseinheit 14 wird Licht über das Kabel 12 zum Kamerahandstück 10 geleitet. Dieses Licht tritt über das Fenster 20 aus. Der Zahnarzt bewegt nun die Kamera so, bis er auf einem durch die Betriebseinheit 14 angesteuerten Monitor denjenigen Bereich des Mundraumes sieht, von dem er eine Aufnahme wünscht. Der Zahnarzt betätigt nun den verformbaren Wandabschnitt 26 so stark, bis eine Aufnahme ausgelöst wird. Hierzu kann die Betriebseinheit 14 eine mit dem Ausgangssignal des Signalverarbeitungskreises 44 beaufschlagte Trigger- oder Diskriminatorstufe enthalten.

Das Auslösen kann die Betriebseinheit 14 z.B. durch ein akustisches Signal bestätigen.

In Abwandlung des oben beschriebenen Ausführungsbeispieles kann man auch andere die Verformung des Wandabschnittes 26 messende Verformungssensoren verwenden, insbesondere Dehnungsmeßstreifen. Der Signalverarbeitungskreis 44 wird dann entsprechend abgeändert.

## Patentansprüche

1. Dentales Handstück, insbesondere intraorales Kamera-Handstück, mit einem Gehäuse (16), innerhalb dessen mindestens eine Arbeitskomponente angeordnet ist, bei dem das Gehäuse (16) mindestens einen elastisch verformbaren Wandabschnitt (26) aufweist, der mit einem Verformungssensor (34) zusammenarbeitet und sich in Umfangsrichtung erstreckt, wobei der Verformungssensor (34) einen Dehnungsmeßstreifen oder eine Piezofolie umfasst und die Umfangserstreckung des verformbaren Wandabschnittes (26) im wesentlichen 360° beträgt, **dadurch gekennzeichnet, dass**
der Dehnungsmeßstreifen oder die Piezofolie eine Ausnehmung (32) überdeckt, die auf der Außenseite einer Stützhülse (28) vorgesehen ist und axial mit dem verformbaren Wandabschnitt (26) fluchtet.

2. Handstück nach Anspruch 1, **dadurch gekennzeichnet,**
**dass** der elastisch verformbare Wandabschnitt (26) kleinere Wandstärke aufweist als nicht verformbare Wandabschnitte des Gehäuses (16) und dass das Gehäuse (16) aus einem elastischen Material gefertigt ist.

3. Handstück nach Anspruch 2, **dadurch gekennzeichnet, dass** das Gehäuse (16) ein Kunststoff-Formteil ist.

4. Handstück nach einem der Ansprüche 1 bis 3, **dadurch**
**gekennzeichnet, dass** hinter dem ausgedehnten verformbaren Wandabschnitt (26) mehrere verformungssensoren (34) angeordnet sind.

5. Handstück nach einem der Ansprüche 1 bis 4, **dadurch**
**gekennzeichnet, dass** der Verformungssensor (34) mit einem Signalverarbeitungskreis (44) verbunden ist, der ein Impedanz- und/oder Amplituden- und/oder Frequenzumgesetztes Signal erzeugt.

6. Handstück nach einem der Anspruche 1 bis 5, **dadurch**
**gekennzeichnet, dass** in Abhängigkeit vom Ausgangssignal des Verformungssensors (34) ein Triggerkreis ausgelöst wird.

## Claims

1. Dental handpiece, in particular intra-oral camera handpiece, with a housing (16), within which at least one working component is arranged, in which the housing (16) has at least one elastically deformable wall section (26), which works with a deformation sensor (34) and extends in a circumferential direction, the deformation sensor (34) including a strain gauge or a piezo foil, and the circumferential extent of the deformable wall section (26) being essentially 360°,
**characterized in that** the strain gauge or piezo foil covers a recess (32), which is provided on the outside of a supporting sleeve (28) and is aligned axially with the deformable wall section (26).

2. Handpiece according to Claim 1, **characterized in that** the elastically deformable wall section (26) has less wall thickness than non-deformable wall sections of the housing (16), and that the housing (16) is manufactured from an elastic material.

3. Handpiece according to Claim 2, **characterized in that** the housing (16) is a plastic preformed part.

4. Handpiece according to any one of Claims 1 to 3, **characterized in that** behind the extended deformable wall section (26), multiple deformation sensors (34) are arranged.

5. Handpiece according to any one of Claims 1 to 4, **characterized in that** the deformation sensor (34) is connected to a signal processing circuit (44), which generates an impedance-converted, amplitude-converted and/or frequency-converted signal.

6. Handpiece according to any one of Claims 1 to 5, **characterized in that** depending on the output signal of the deformation sensor (34), a trigger circuit is triggered.

## Revendications

1. Pièce à main dentaire, en particulier pièce à main de caméra intraorale, avec un boîtier (16) à l'intérieur duquel est disposé au moins un composant de travail, dans laquelle le boîtier (16) présente au moins une portion de paroi déformable élastiquement (26) qui coopère avec un capteur de déformation (34) et s'étend en direction circonférentielle, le capteur de déformation (34) comprenant une jauge extensométrique ou une feuille piézoélectrique et l'extension circonférentielle de la portion de paroi déformable (26) étant essentiellement de 360°, **caractérisée en ce que** la jauge extensométrique ou la feuille piézoélectrique recouvre un évidement (32) qui est prévu sur le côté extérieur d'une douille de soutien (28) et est aligné axialement avec la portion de paroi déformable (26).

2. Pièce à main selon la revendication 1, **caractérisée en ce que** la portion de paroi déformable élastiquement (26) présente une épaisseur de paroi plus faible que des portions de paroi non déformables du boîtier (16) et que le boîtier (16) est réalisé dans un matériau élastique.

3. Pièce à main selon la revendication 2, **caractérisée en ce que** le boîtier (16) est une pièce moulée en matière plastique.

4. Pièce à main selon une des revendications 1 à 3, **caractérisée en ce que** plusieurs capteurs de déformation (34) sont disposés derrière la portion de paroi déformable (26) étendue.

5. Pièce à main selon une des revendications 1 à 4, **caractérisée en ce que** le capteur de déformation (34) est relié à un circuit de traitement de signaux (44) qui génère un signal converti en impédance et/ou amplitude et/ou fréquence.

6. Pièce à main selon une des revendications 1 à 5, **caractérisée en ce qu'**un circuit de déclenchement est déclenché en fonction du signal de sortie du capteur de déformation (34).
